# EUROPEAN PATENT APPLICATION

(11) **EP 4 559 456 A1**
(43) Date of publication of application: **28.05.2025**
(21) Application number: 24214077.0
(22) Date of filing: 20.11.2024
(51) Int. Cl.: A61K 9/20

(54) **A MODIFIED RELEASE TABLET OF BRIVARACETAM**

(30) Priority: 22.11.2023 TR 202315501
(71) Applicant: Sanovel Ilac Sanayi ve Ticaret A.S., Sisli - Istanbul (TR)
(72) Inventor: SUNEL, Fatih, Istanbul (TR); BILGEHAN ATAK, Fadime, Istanbul (TR); ATAMAN, Seval, Istanbul (TR); DERELI, Selin, Istanbul (TR)
(74) Representative: Genç Ilhan, Oznur

(57) **Abstract**

The present invention relates to a modified release tablet formulation comprises brivaracetam and at least one pharmaceutically acceptable excipient wherein the amount of brivaracetam is 20.0% to 55.0% by weight in the total composition and at least one matrix agent. Furthermore, the tablet is also obtained by using an effective process which is simple, rapid, cost effective, time-saving and industrially convenient process.

## Description

### Field of the Invention

The present invention relates to a modified release tablet formulation comprises brivaracetam and at least one pharmaceutically acceptable excipient wherein the amount of brivaracetam is 20.0% to 55.0% by weight in the total composition and at least one matrix agent. Furthermore, the tablet is also obtained by using an effective process which is simple, rapid, cost effective, time-saving and industrially convenient process.

### Background of the Invention

The chemical name of brivaracetam is (2S)-2-[(4R)-2-oxo-4-propyl-pyrrolidin-1-yl]butanamide and its chemical structure is shown in the Formula I.

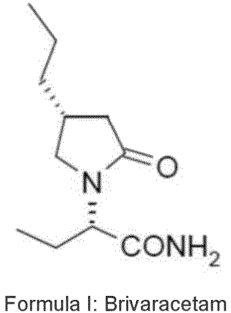

The European Medicines Agency (EMA) and the US Food and Drug Administration (FDA) approved brivaracetam as an adjunctive therapy drug for the treatment of partial seizures with or without secondary generalized seizures of epilepsy patients of 16-year-old and older, under the trade name Briviact^{®}. The existing commercially-available preparations comprise a brivaracetam tablet, a brivaracetam oral liquid and a brivaracetam injection, wherein the brivaracetam tablet has a plurality of specifications such as 10 mg, 25 mg, 50 mg, 75 mg and 100 mg, and both the brivaracetam oral liquid and the brivaracetam injection have a specification of 10 mg/mL.

Brivaracetam is highly soluble and is Class I according to the Biopharmaceutical Classification System (BCS). The active substance is a white to off-white non-hygroscopic crystalline solid. Additionally, brivaracetam is an extremely viscous compound (adhesive capacity). Brivaracetam has short half-life and high-water solubility. These characteristics are ideal to develop modified release pharmaceutical compositions which invariably will offer various advantages over conventional immediate release drug composition such as constant therapeutic plasma concentration of Brivaracetam over prolonged periods, reduced number of doses per day or week, reduced adverse effects and improved patient compliance and convenience.

A modified release formulation would be particularly desirable for administration in some patients. A prolonged release formulation could be advantageously used in order to reduce the difference between plasmatic Cₘₐₓ and Cₘᵢₙ and consequently to lower sides effects. Moreover, a prolonged release formulation improves the patient's compliance as the administration frequency could be reduced.

Patent WO 2009/144286 discloses a pharmaceutical composition in the form of a film coated tablet comprising Brivaracetam and, as excipient within the core of the tablet, 5% to 80% per weight of at least one hydrophilic matrix agent for prolonged release.

US2013039957 discloses a controlled release pharmaceutical compositions comprising Brivaracetam or its pharmaceutically acceptable derivatives thereof.

There still remains a need in the art to provide an improved a modified release tablet comprising brivaracetam. In the present invention, the modified release tablet is created to overcome the above problems and provides additional advantages to the relevant field of art. Other advantages and embodiments of the present invention will be clarified in the following description.

### Detailed Description of the Invention

The main object of the present invention is to control the release of brivaracetam and ensure a long-term effective therapeutic effect when administered to the patient which overcomes the above-described problems in the prior art and have additive advantages over them.

Another object of the present invention is to provide a modified release tablet comprising brivaracetam with excellent properties, such as compressibility, flowability, homogeneity, content uniformity and stability and the desired dissolution profile.

Another object of the present invention is to provide a process for preparing a modified release tablet comprising brivaracetam. The process is wet granulation or hot melt extrusion. It is a simple, rapid, cost effective, time-saving, and industrially convenient method.

The term "modified release" refers to any pharmaceutical formulation that maintain constant levels of a drug in the patient's bloodstream by releasing the drug over an extended period of time. Modified release is formulated to release the active ingredient gradually and predictably over a 12-hour to 24-hour period. Modified release is selected from the group comprising controlled release, sustained release, delayed release, extended release, repeat action system or mixtures thereof.

Content uniformity is an important parameter in tablet formulation. Especially the high sticky behavior of the active substance caused content uniformity problems in formulations. The problem with flowability negatively affects the dissolution profile. We have found that these problems are overcome when using brivaracetam at the specified ranges. We also used matrix agents for the desired release profile.

According to one embodiment of this invention, a modified release tablet formulation comprises brivaracetam and at least one pharmaceutically acceptable excipient wherein the amount of brivaracetam is 20.0% to 55.0% by weight in the total composition and at least one matrix agent.

According to one embodiment of this invention, the tablet does not comprise an enteric coating layer.

According to one embodiment of this invention, the amount of brivaracetam is 20.0% to 30.0% by weight or 40.0% to 55.0% by weight in the total composition.

The modified release effect of the tablet is achieved using suitable matrix agents. Suitable matrix agents are selected from the group comprising hydroxypropylmethyl cellulose (high viscosity), xanthan gum, carnauba wax, hydroxyethyl cellulose, hydroxypropyl cellulose, hydroxyethyl methyl cellulose, methylcellulose, methylacrylates, polyethylene glycols, polysaccharides, acrylic acid or its derivatives, glycerol monostearate, polyamides, polycarbonates, polyalkylene, polyalkylene oxides, polyvinyl alcohols, polyvinyl ethers, polymers of acrylic and methacrylic esters, polylactides, poly(fumaric acid) or mixtures thereof.

According to one embodiment of this invention, the matrix agent is hydroxypropylmethyl cellulose (high viscosity) or xanthan gum or carnauba wax or mixtures thereof.

According to one embodiment of this invention, the matrix agent is xanthan gum or carnauba wax or mixtures thereof.

According to one embodiment of this invention, the matrix agent is hydroxypropylmethyl cellulose (high viscosity).

According to one embodiment of this invention, the amount of matrix agent is 15.0% to 40.0% by weight in the total composition.

We found that there is no flowability problem when the active ingredient is used in the particle sizes specified below.

As used here in, 'particle size' means the cumulative volume size distribution as tested by any conventionally accepted method such as the laser diffraction method (i.e. Malvern analysis). The term d (0.1) means, the size at which 10% by volume of the particles are finer and d (0.5) means the size at which 50% by volume of the particles are finer and d (0.9) means the size at which %90 by volume of the particles is finer.

According to one embodiment of this invention, brivaracetam has a d (0.9) particle size between 30 µm to 450 µm.

According to one embodiment of this invention, brivaracetam has a d (0.9) particle size between 50 µm to 90 µm or between 95 µm to 125 µm or between 126 µm to 138 µm or between 139 µm to 152 µm or between 153 µm to 167 µm or between 168 µm to 183 µm or between 184 µm to 198 µm or between 203 µm to 218 µm or between 219 µm to 234 µm or between 237 µm to 253 µm or between 257 µm to 268 µm or between 269 µm to 278 µm or between 283 µm to 298 µm or between 304 µm to 345 µm or between 348 µm to 390 µm or between 395 µm to 445 µm.

According to one embodiment of this invention, brivaracetam has a d (0.1) particle size between 1 µm to 40 µm or between 4 µm to 34 µm or between 6 µm to 25 µm.

According to one embodiment of this invention, brivaracetam has a d (0.5) particle size between 5 µm to 85 µm or between 13 µm to 76 µm or between 22 µm to 65 µm or between 27 µm to 58 µm.

According to one embodiment of this invention, the modified release tablet further comprising at least one pharmaceutically acceptable excipient which is selected from the group comprising diluents, lubricants or mixtures thereof.

Suitable diluents are selected from the group comprising microcrystalline cellulose, lactose spray dried, lactose anhydrous, lactose, lactose monohydrate, corn starch, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

According to an embodiment of the present invention, the diluents are microcrystalline cellulose or lactose or lactose spray dried or mixtures thereof.

According to an embodiment of the present invention, the amount of diluents is 20.0% to 40.0% by weight in the total composition. Preferably, it is 25.0% to 35.0% by weight.

Suitable lubricant is selected from the group comprising talc, sodium stearyl fumarate, magnesium stearate, potassium stearate, stearic acid, sodium chloride, sodium benzoate, sodium acetate, sodium oleate, polyethylene glycols or mixtures thereof.

According to one embodiment of the present invention, the lubricant is magnesium stearate or sodium stearyl fumarate or mixtures thereof.

According to one embodiment of the present invention, the amount of lubricant is between 0.1% and 3.0% by weight in the total formulation.

According to an embodiment of the present invention, binder can be used in the tablet. Suitable binders are selected from the group comprising carbomer, hydroxypropyl methyl cellulose (low viscosity), hydroxypropyl cellulose, sodium carboxymethyl cellulose, polyvinylpyrrolidone, sodium carboxymethyl cellulose, polyethylene glycol, pregelatinized starch, sodium alginate, carboxy methyl cellulose, methyl cellulose, polymethacrylates, methacrylate polymers, polysaccharides, poloxamer, polyoxyethylene-alkyl ether, polydextrose, polyethylene oxide or mixtures thereof.

According to an embodiment of the present invention, the binder is carbomer or hydroxypropyl methyl cellulose (low viscosity) or mixtures thereof.

According to an embodiment of the present invention, the binder is carbomer.

According to an embodiment of the present invention, the amount of binders is 2.0% to 10.0% by weight in the total composition.

According to one embodiment of the present invention, the modified release tablet comprises;
- Brivaracetam
- Carbomer
- Hypromellose (Low viscosity)
- Hypromellose (High viscosity)
- Lactose spray dried
- Microcrystalline cellulose
- Mg Stearate
- Purified water*

According to one embodiment of the present invention, the modified release tablet comprises;
- Brivaracetam
- Lactose
- Carnauba wax
- Xanthan gum
- Magnesium stearate

We found that the brivaracetam is extremely prone to sticking and picking during the preparation process, which brings great problems to the appearance and content uniformity of the final product. We have seen that these problems are overcome when wet granulation or hot melt extrusion is used.

According to an embodiment of the present invention, the modified release tablet comprising brivaracetam is prepared by hot melt extrusion.

According to an embodiment of the present invention, the modified release tablet comprising brivaracetam is prepared by wet granulation.

### Example 1: The tablet prepared by hot melt

| **Ingredients** | **% by weight** | **% by weight** |
|---|---|---|
| Brivaracetam | 20.0 - 55.0 | 50.0 |
| Lactose | 20.0 - 40.0 | 28.0 |
| Carnauba wax | 5.0 - 20.0 | 10.0 |
| Xanthan gum | 5.0 - 20.0 | 10.0 |
| Magnesium stearate | 0.1 - 3.0 | 2 |
| **Total** | **100** | **100** |
| **Film coating** | | |

A process for example 1;
a) Melting and sieving Carnauba wax,
b) Granulating weighed and sieved Brivaracetam and xanthan gum with the melt,
c) Sieving granulates through an appropriate sieve,
d) Adding weighed and sieving lactose and magnesium stearate to granulate and mix.
e) Compressing the homogenous mixture into tablet,
f) Coating the tablets with the PVA or HPMC based film coating material.

### Example 2: The tablet prepared by wet granulation

| **Ingredients** | **% by weight** | **% by weight** |
|---|---|---|
| Brivaracetam | 20.0 - 55.0 | 25.0 |
| Carbomer | 2.0 - 6.0 | 4.75 |
| Hypromellose (Low viscosity) | 0.5 - 4.0 | 1 |
| Hypromellose (High viscosity) | 20.0 - 40.0 | 36.25 |
| Lactose spray dried | 1.0 - 10.0 | 5.0 |
| Microcrystalline cellulose | 20.0 - 35.0 | 27.5 |
| Mg Stearate | 0.1 - 3.0 | 0.5 |
| Purified water* | q.s | q.s |
| **Total** | **100** | **100** |
| **Film coating** | | |

| | | |
|---|---|---|
| *: sufficient quantity | | |

A process for example 2;
a) Weighing and sieving Brivaracetam, Carbomer, Microcrystalline cellulose, Hypromellose (low viscosity) and mixing for 15 minutes
b) Adding water to the resulting mixture to granulate,
c) Grinding the dried granule up to the desired particle size,
d) Adding Hydroxypropyl methylcellulose (High viscosity) to the grinded granules and mixing,
e) Adding Lactose to the mixture and mixing,
f) Adding magnesium stearate to the mixture and mixing,
g) Compressing the homogenous mixture into tablet,
h) Coating the tablets with the PVA or HPMC based film coating material.

## Claims

1. A modified release tablet formulation comprises brivaracetam and at least one pharmaceutically acceptable excipient wherein the amount of brivaracetam is 20.0% to 55.0% by weight in the total composition and at least one matrix agent.

2. The modified release tablet formulation according to claim 1, wherein the tablet does not comprise an enteric coating layer.

3. The modified release tablet formulation according to claim 1, wherein the amount of brivaracetam is 20.0% to 30.0% by weight or 40.0% to 55.0% by weight in the total composition.

4. The modified release tablet formulation according to claim 1, wherein matrix agents are selected from the group comprising hydroxypropylmethyl cellulose (high viscosity), xanthan gum, carnauba wax, hydroxyethyl cellulose, hydroxypropyl cellulose, hydroxyethyl methyl cellulose, methylcellulose, methylacrylates, polyethylene glycols, polysaccharides, acrylic acid or its derivatives, glycerol monostearate, polyamides, polycarbonates, polyalkylene, polyalkylene oxides, polyvinyl alcohols, polyvinyl ethers, polymers of acrylic and methacrylic esters, polylactides, poly(fumaric acid) or mixtures thereof.

5. The modified release tablet formulation according to claim 1 or 4, wherein the matrix agent is hydroxypropylmethyl cellulose (high viscosity) or xanthan gum or carnauba wax or mixtures thereof.

6. The modified release tablet formulation according to claim 1 or 5, wherein the matrix agent is xanthan gum or carnauba wax or mixtures thereof.

7. The modified release tablet formulation according to claim 1 or 5, wherein the matrix agent is hydroxypropylmethyl cellulose (high viscosity).

8. The modified release tablet formulation according to claim 1 or 5, wherein the amount of matrix agent is 15.0% to 40.0% by weight in the total composition.

9. The modified release tablet formulation according to claim 1, wherein brivaracetam has a d (0.9) particle size between 30 µm to 450 µm.

10. The modified release tablet formulation according to claim 1, wherein further comprising at least one pharmaceutically acceptable excipient which is selected from the group comprising diluents, lubricants or mixtures thereof.

11. The modified release tablet formulation according to claim 10, wherein diluents are selected from the group comprising microcrystalline cellulose, lactose spray dried, lactose anhydrous, lactose, lactose monohydrate, corn starch, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

12. The modified release tablet formulation according to claim 11, wherein the diluents are microcrystalline cellulose or lactose or lactose spray dried or mixtures thereof.

13. The modified release tablet formulation according to claim 10, wherein further binder is used in the tablet.

14. The modified release tablet formulation according to claim 10, wherein the tablet is prepared by hot melt extrusion.

15. The modified release tablet formulation according to claim 10, wherein the tablet is prepared by direct compression.
